# EUROPÄISCHE PATENTSCHRIFT

(11) **EP 3 809 951 B1**
(45) Veröffentlichungstag und Bekanntmachung des Hinweises auf die Patenterteilung: **31.07.2024**
(21) Anmeldenummer: 19741970.8
(22) Anmeldetag: 24.06.2019
(51) Int. Cl.: A61B 5/00, A61B 5/0205, A61B 5/024

(54) **MESSVORRICHTUNG**
MEASURING DEVICE
DISPOSITIF DE MESURE

(30) Priorität: 22.06.2018 DE 102018004955
(43) Veröffentlichungstag der Anmeldung: 28.04.2021
(73) Patentinhaber: Flore, Ingo, 44141 Dortmund (DE)
(72) Erfinder: CHO, Ok-Kyung, 58239 Schwerte (DE); KIM, Yoon Ok, 58239 Schwerte (DE)
(74) Vertreter: Schneiders & Behrendt Bochum
(86) Internationale Anmeldenummer: PCT/EP2019/066615
(87) Internationale Veröffentlichungsnummer: WO 2019/243629

(56) Entgegenhaltungen:
- EP-A1- 3 492 005
- WO-A1-2012/013350
- CN-U- 202 128 448
- US-A1- 2007 038 048
- US-A1- 2010 222 652
- US-A1- 2010 234 701
- US-A1- 2014 243 612
- US-A1- 2016 022 213
- US-A1- 2017 296 088
- US-A1- 2018 317 859

## Beschreibung

Die Erfindung betrifft eine multifunktionale Messvorrichtung aufweisend ein Gehäuse mit einer Oberschale und einer Unterschale, welche mittels eines Klappmechanismus gegeneinander bewegbar sind und die miteinander korrespondierende Mulden aufweisen, wobei die Mulden eine von außen zugängliche Kammer zur Aufnahme eines menschlichen Fingers bilden, wobei in der Kammer eine optische Messeinheit mit einem optischen Modul, welches mindestens eine Lichtquelle und mindestens einen Sensor aufweist, angeordnet ist, wobei im oder am Gehäuse Mittel zur Datenauswertung und/oder Datenweiterleitung integriert sind und wobei mindestens eine elektrische Messeinheit mit mindestens zwei Messelektroden vorgesehen ist. Des Weiteren betrifft die Erfindung ein Verfahren zur Durchführung einer Messung unter Verwendung einer solchen Multifunktionalen Messeinrichtung.

Eine solche Messvorrichtung ist beispielsweise aus der WO 2012/013350 A1 bekannt. Tragbare und einfach zu bedienende Multifunktionsmessvorrichtungen für Healthcare- und Medizin-Anwendungen ermöglichen es Anwendern zu Hause und unterwegs, ihren Gesundheitszustand zu überwachen. Je nach Anwendungsgebiet und Zielsetzung können verschiedene Parameter für die Überwachung des Gesundheitszustands des Anwenders relevant sein, beispielsweise die Herzrate, die arterielle Sauerstofffsättigung oder andere aus einem ECG (Elektrokardiogramm) oder Photoplethysmogram abgeleitete Parameter.

Aus der US 2016/0022213 A1 ist ein Fitnesstracker bekannt, mit welchem unterschiedliche Körperparameter erfasst werden können. Der dortige Fitnesstracker wird am Handgelenk getragen und ist daher eher flach ausgestaltet. Demensprechend weist dieser keine zur Aufnahme eines Fingers geeigneten Mulden auf, sondern sieht einen zusätzlichen flachen Klemmadapter vor, um den Finger am Fitnesstracker festzulegen.

In der US 1014/0243612 A1 ist Fingerpulsoximeter mit einem externen über ein Kabel verbundenes Analysegerät offenbart. Am Analysegerät kann weitere Sensorik, angeordnet sein. Insgesamt ist dieser mehrteilige eher kompliziert zu bedienen.

Zur Messung des Pulses und Sauerstoffsättigung werden häufig eingangs genannte Messvorrichtungen, sogenannte "Fingerpulsoximeter", eingesetzt.

Sollen aber weitere und mehrere unterschiedliche physiologische Parameter bestimmt werden, muss häufig auf verschiedene Einzelgeräte zurückgegriffen werden. Dies ist für den Anwender sowohl in Bezug auf die Anschaffung als auch die Nutzung unpraktisch. Außerdem ist es bei Verwendung verschiedener Geräte aufwändig, die gemessenen Daten zu integrieren und zu kombinieren.

Es ist daher Aufgabe der Erfindung, eine kompakte, handliche Messvorrichtung dahingehend weiterzubilden, dass die Bestimmung einer Vielzahl nicht-invasiv bestimmbarer Parameter durch die Messvorrichtung ermöglicht wird. Darüber hinaus soll ermöglicht werden, mithilfe statistischer Verfahren (z.B. multivariate Verfahren) und/oder Verfahren des maschinellen Lernens (z.B. neuronale Netze, auch in Zusammenhang mit Deep Learning) weitere Parameter zu bestimmen, die der nicht-invasiven Messung normalerweise nicht direkt zugänglich sind.

Zur Lösung der Aufgabe schlägt die Erfindung eine Messvorrichtung nach Anspruch **1** vor. Weitere Ausführungsformen werden von abhängigen Ansprüchen **2-12** offenbart. Zudem umfasst die Erfindung ein Verfahren nach Anspruch **13**. Eine weitere Ausführungsform wird in Anspruch **14** offenbart.

Durch die elektrische Messeinheit können zusätzlich zu den optischen Messungen auch elektrische Messungen, wie beispielsweise eine Bioimpedanzmessung oder Elektrokardiogramm-Messung (ECG), durchgeführt werden. Zudem können die zusätzlichen elektrischen Messergebnisse mit den optischen Messergebnissen kombiniert werden. Die Messvorrichtung verfügt über mehrere optische Sensoren, die entlang des Fingers platziert sind. Da der Abstand der Sensoren bekannt ist, kann man aus der Zeitdifferenz, mit der die unterschiedlichen optischen Sensoren die Änderung der Gewebeabsorption aufgrund der Pulsation des arteriellen Blutes detektieren, einen Indikator für die Pulswellengeschwindigkeit ohne zusätzliche ECG-Messung berechnen. Erfindungsgemäß ist der Abstand der optischen Sensoren im Messsystem möglichst groß gewählt, damit die zu messende Zeitdifferenz ebenfalls möglichst groß wird. Hierzu werden weiter unten noch Ausführungen gemacht.

Eine Weiterbildung der Erfindung sieht vor, dass mindestens eine Temperaturmessvorrichtung am Gehäuse angeordnet ist. Mittels der Temperaturmesseinheit kann beispielsweise die Fingertemperatur des Anwenders festgestellt werden, und die entsprechenden Messdaten können in die Auswertung mit einbezogen werden.

Eine bevorzugte Ausführungsform der Erfindung sieht vor, dass mindestens ein weiterer optischer Sensor und/oder eine weitere Lichtquelle gegenüber dem optischen Modul angeordnet sind. Durch die Anordnung eines weiteren Sensors oder einer weiteren Lichtquelle können neben der Reflexionsmessung durch den optischen Sensor und die Lichtquelle in dem optischen Modul auch Transmissionsmessungen durchgeführt werden und die so gewonnen Messdaten zur Analyse hinzugezogen werden. Durch die Messung der Reflexion und Transmission ist die Bestimmung physiologischer Parameter für unterschiedliche Gewebebereiche (oberflächennahe bzw. tiefer liegende Gewebeschichten) möglich. Unterschiedliche Gewebebereiche sind unterschiedlich venös bzw. arteriell durchblutet. Die Kombination von Messwerten aus venös bzw. arteriell durchblutetem Gewebe ermöglicht Rückschlüsse auf wichtige Stoffwechselparameter.

Es ist zweckmäßig, wenn der Klappmechanismus mit einem Rückstellmechanismus versehen ist. Hierzu kann beispielsweise ein Federmechanismus dienen. Nachdem die beiden Schalen auseinander gedrückt wurden und der Finger eingelegt wurde, schließen die beiden Schalen wieder selbsttätig und klemmen den Finger zwischen sich ein. Mittels des Rückstellmechanismus kann der Druck der Klemmung auf den gewünschten Wert reproduzierbar voreingestellt sein. Der Anpressdruck des Fingergewebes an den entsprechenden Sensoren beeinflusst die Messung. Dieser Parameter sollte daher (zumindest näherungsweise) definiert sein.

Eine bevorzugte Ausführungsform sieht vor, dass zur Datenauswertung ein Mikrocontroller im Gehäuse angeordnet ist. Mittels des Mikrocontrollers kann die Datenauswertung direkt in der Messvorrichtung durchgeführt werden.

Eine Weiterbildung der Erfindung sieht vor, dass die Mittel zur Datenweiterleitung über eine Drahtlosschnittstelle verfügen. Diese kann die Daten weiterleiten und der Anwender kann die Daten auf einem externen Gerät, beispielsweise einem Smartphone oder einer Smartwatch, ansehen, speichern und weiterverarbeiten. Auch die Steuerung der Messvorrichtung über ein solches externes Gerät ist möglich.

Besonders zweckmäßig ist es, dass Vorrichtungen zur Positionierung der einzelnen Finger vorgesehen sind, derart dass sich die Finger beim Messvorgang immer in der gleichen Position befinden. Hierdurch kann sichergestellt werden, dass die Finger für die Durchführung der Messung richtig positioniert werden. Die speziellen Ausführungen der jeweiligen Vorrichtungen werden weiter unten beschrieben.

In einer Ausführungsform der Messvorrichtung ist vorgesehen, dass ein Beschleunigungssensor und/oder ein Gyroskop integriert sind. Hierdurch können Bewegungen der Messvorrichtung bei der Datenauswertung berücksichtigt werden oder der Anwender darauf hingewiesen werden, dass die Messwerte aufgrund zu ausgeprägter Bewegung der Messvorrichtung eventuell nicht korrekt sind.

Es kann auch sinnvoll sein, weitere Sensoren zur Messung des Luftdrucks, der Luftfeuchtigkeit und/oder der Umgebungstemperatur zu integrieren. Hierdurch kann der Einfluss der Umgebungsparameter bei der Messdatenanalyse einbezogen werden.

Vorteilhaft ist zudem, wenn Drucksensoren zu Messung des Anpressdrucks des Fingers integriert sind. Hierdurch kann beispielsweise eine Fehlfunktion des Rückstellmechanismus detektiert werden. Die Messungen der Drucksensoren können aber auch zur Korrektur von druckabhängigen Messwerten verwendet werden. Außerdem kann es je nach Anwendungszweck nützlich sein, die durch die Pulsation des Blutes im Finger hervorgerufene, dem Anpressdruck überlagerte Druckänderung als eigenständiges Messsignal auszuwerten und daraus physiologische Parameter zu abzuleiten.

Weiterhin kann es zweckmäßig sein, dass externe Anschlüsse für weitere externe Sensorik am Gehäuse angeordnet sind. An den Anschlüssen können auch externe Sensoren angeschlossen werden, sodass diese beispielsweise an anderen Körperstellen als der Hand angelegt werden können.

Im Folgenden werden Ausführungsbeispiele der Erfindung anhand von Zeichnungen näher erläutert. Es zeigen:
- Figur 1 a-f:: Eine erfindungsgemäße Messvorrichtung in verschiedenen Ansichten im zusammengeklappten Zustand;
- Figur 2 a-d:: die erfindungsgemäße Messvorrichtung aus Figur 1 a-f in verschiedenen Ansichten im aufgeklappten Zustand;
- Figur 3 a-b:: schematisch ein erfindungsgemäße Messvorrichtung bei der Benutzung durch einen Anwender;
- Figur 4:: schematisch einen Verfahrensablauf bei einer Messung mit einer erfindungsgemäßen Messvorrichtung;
- Figur 5:: schematisch die Messdatenerfassung und -verarbeitung.

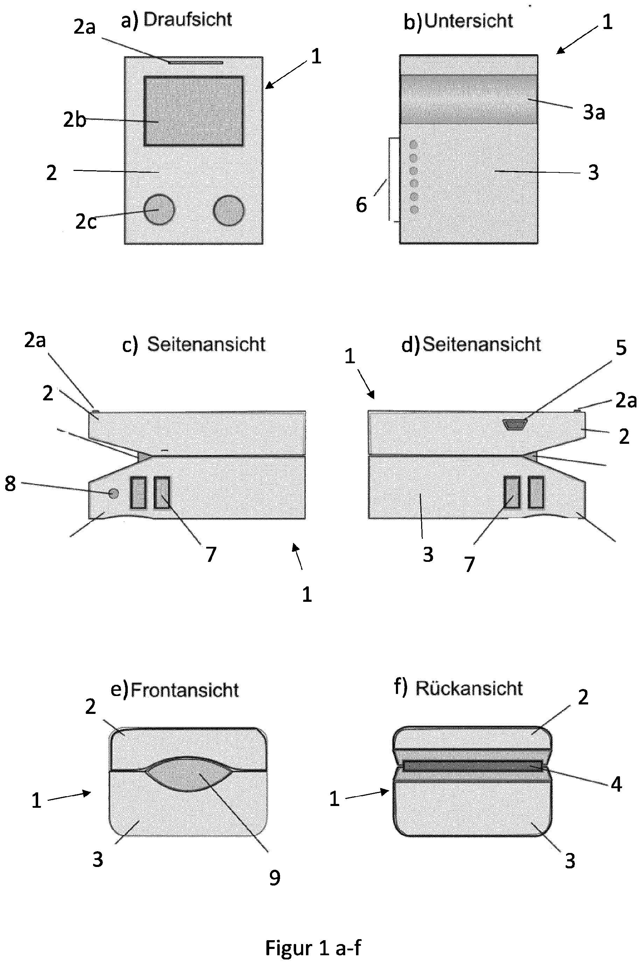

Eine erfindungsgemäße Messvorrichtung ist in den Figuren 1 a-f anhand einer konkreten Realisierung dargestellt. Die Darstellung beschränkt sich hier auf die äußerlichen Merkmale der Messvorrichtung, weitere mechanische Aspekte und die Merkmale des inneren Teils der Messvorrichtung werden weiter unten beschrieben.

Das Gehäuse ist allgemein mit dem Bezugszeichen 1 bezeichnet. Die wesentlichen Merkmale des Gehäuses 1 der Messvorrichtung sind wie folgt:
- Das Gehäuse 1 besteht aus einer Oberschale 2 und einer Unterschale 3, welche durch Zusammendrücken eines Klappmechanismus 4 an der Hinterseite des Gehäuses 1 an der Vorderseite auseinanderbewegt werden können.
- Um das Greifen der Messvorrichtung beim Zusammendrücken an der Hinterseite des Gehäuses 1 für den Nutzer zu erleichtern, befindet sich an der Oberschale 2 ein kleiner Steg 2a und an der Unterschale eine Vertiefung 3a.
- Die Oberschale 2 enthält eine Anzeige 2b sowie die Bedienelemente 2c. An der Seite der Oberschale 2 befinden sich optional ein Konnektor 5 für eine externe Schnittstelle (z.B. USB) sowie Metallkontakte 6 beispielsweise zur Aufladung der Messvorrichtung in einer Andockstation.
- An den Seiten der Unterschale 3 befinden sich jeweils zwei Elektroden 7 für Bioimpedanz- und ECG-Messungen. An einer der Seiten des Gehäuses befindet sich zusätzlich ein Temperatursensor 8.
- Die Frontansicht (Figur 1e) zeigt eine Kammer 9, in welche ein Finger im aufgeklappten Zustand eingelegt werden kann. In der Kammer 9 befinden sich mindestens eine optische Messeinheit und optional noch weitere Messeinheiten.

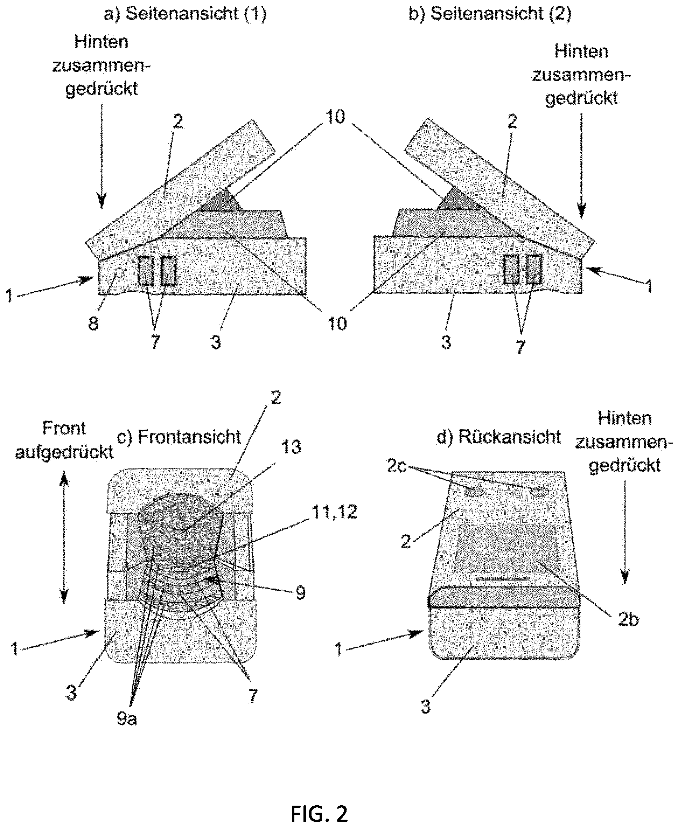

Das Zusammendrücken der Oberschale 2 und Unterschale 3 an der Hinterseite der Messvorrichtung aus den Figuren 1 a-f ist in den Figuren 2 a-d dargestellt. Wenn sich Oberschale 2 und Unterschale 3 an der Vorderseite des Gehäuses 1 geöffnet haben, kann ein Finger in die Messvorrichtung eingelegt werden. Ober- und Unterschale 2, 3 sind über einen Federmechanismus, der als Klappmechanismus 4 dient, miteinander verbunden.

Wird der Finger eingelegt und werden die am hinteren Teil der Messvorrichtung zusammengedrückte Ober- und Unterschale 2, 3 losgelassen, dann ziehen sich Ober- und Unterschale 2, 3 zusammen und über den Federmechanismus wird ein definierter Druck auf den Finger ausgeübt. Andere Mechanismen, die ein Öffnen der Messvorrichtung zum Einlegen des Fingers sowie die Ausübung eines definierten Drucks auf den Finger ermöglichen, sind aber ebenso möglich und beeinträchtigen nicht den Kern der Erfindung.

Figuren 2 a-d zeigen die Messvorrichtung aus den Figuren 1 a-f im aufgeklappten Zustand. Da sich Ober- und Unterschale 2, 3 bei eingelegtem Finger nicht vollständig in ihre Ausgangsposition zurückbewegen können, befinden sich sowohl an der Oberschale 2 als auch an der Unterschale 3 seitlich angebrachte Wände 10, die den Einfall von Umgebungslicht reduzieren. Zwischen diesen Wänden 10 befinden sich die Mulden an der Ober- und Unterschale 2, 3, die in Figur 2c dargestellt sind. Die Mulden bilden die Kammer 9 zur Aufnahme eines Fingers. Die wesentlichen Eigenschaften der inneren Kammer 9 sind wie folgt:
- Die Kammer 9 ist sowohl oben als auch unten gekrümmt. Die Krümmung der Kammer spiegelt dabei einerseits die Krümmung des menschlichen Fingers wider. Andererseits wird damit auch erreicht, dass der eingelegte Finger eine stabile Messposition hat.
- Am hinteren Ende der Kammer 9 befindet sich eine Rückwand, bis zu der der Finger hineingeschoben werden muss. Dadurch hat der Finger eine festgelegte Endposition.
- Es sind mehrere Arten von Messeinheiten in die Kammer integrierbar, die für optische, elektrische und Temperatur-Messungen verwendet werden können.
- Die Teile der Auflagefläche, die keine Messeinheiten beinhalten, sind mit einem weichen Material 9a ausgekleidet, damit scharfe Kanten vermieden werden und sich der Komfort des Nutzers erhöht.

Auf die hier verwendeten Sensoren sowie deren Positionierung wird im folgenden Abschnitt genauer eingegangen.
- Der untere Teil der Kammer 9 enthält eine optische Messeinheit in Form eines optischen Moduls 11 mit Lichtquellen 12 sowie optischen Sensoren. Das optische Modul 11 ist unterhalb des distalen Fingerglieds platziert. Mithilfe der optischen Sensoren sowie den Lichtquellen 12 im optischen Modul 11 kann die diffuse Reflexion des Fingergewebes gemessen werden.
- Bei den Lichtquellen 12 des optischen Moduls 11 kann es sich beispielsweise um LEDs verschiedener Wellenlängen handeln. Zur Messung der diffusen Reflexion des Fingers können z.B. eine oder mehrere Photodioden im optischen Modul 11 verwendet werden. Das Modul 11 kann über einen zusätzlichen Temperatursensor verfügen, der auch Informationen über die Temperatur der Lichtquellen 12 innerhalb des Moduls 11 liefern kann.
- Ein weiterer optischer Sensor 13 ist im oberen Teil der Fingerauflage als Teil der optischen Messeinheit platziert. Mithilfe dieses zusätzlichen Sensors 13 kann Transmission durch den Finger gemessen werden, wobei das durchstrahlte Gewebe ein im Vergleich zum unteren Sensor anderes ist.
- Messelektroden 7 befinden sich sowohl in der inneren Kammer 9 als auch auf den Außenseiten des Geräts. In der konkreten Realisierung werden Edelstahlelektroden verwendet, andere Materialen sind jedoch ebenfalls möglich.
- Mithilfe der innen in der Kammer 9 und außen am Gehäuse 1 angeordneten Messelektroden 7 kann ein ECG zwischen Fingern der linken und rechten Hand gemessen werden. Ebenso sind verschiedene Bioimpedanzmessungen möglich. Durch Kombination verschiedener Messelektroden 7 können beispielsweise in der dargestellten Realisierung folgende Bioimpedanzmessungen durchgeführt werden:
   ∘ Bioimpedanzmessung zwischen linkem und rechtem Zeigefinger.
   ∘ Bioimpedanzmessung zwischen rechtem Zeigefinger und rechtem Daumen.
- An der Außenseite befindet sich ein Temperatursensor 8, der bei Kontakt mit einem Finger die Fingertemperatur misst. Beispielsweise kann der Temperatursensor 8 auch in die Kammer 9 integriert sein.

Die Reihenfolge und relative Positionierung der Sensoren kann der Positionierung in den Figuren 1 a-f und 2 a-d entsprechen, aber auch für spezielle Anwendungen angepasst werden. Z.B. könnte das optische Modul 11 auch zwischen den beiden Elektroden 7 der inneren Fingerauflage positioniert werden.

Die multifunktionale Messvorrichtung ist batterie- oder akkubetrieben und weist mehrere Messeinheiten auf. Bei Varianten der Messvorrichtung ohne Andockstation werden externe Schnittstellen direkt in die Messvorrichtung integriert. Die Grundform einer Ausführungsform der Messvorrichtung ist rechteckig (bspw. Länge x Breite x Höhe (ca.): 7 cm x 4.5 cm x 3.5 cm, Gewicht: 85 g), die genaue Form weicht jedoch aus ergonomischen und funktionalen Gründen von einem Rechteck ab. Beispielsweise muss die Messvorrichtung aufklappbar sein und die Ecken des Gehäuses 1 sind zur Vermeidung scharfer Kanten abgerundet.

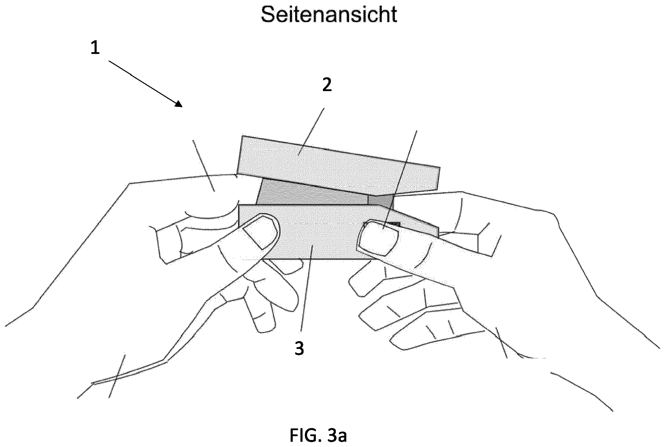

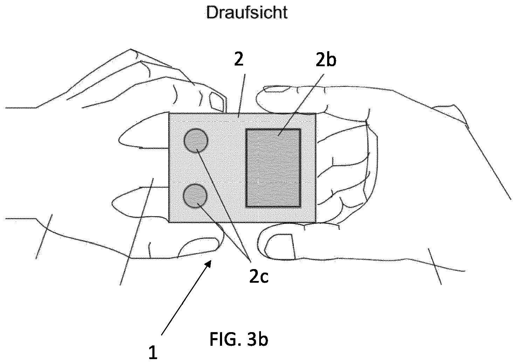

Die Figuren 3 a-b zeigen einen beispielhaften Messvorgang. Die Messvorrichtung wird vom Nutzer mit den Händen festgehalten und der linke Zeigefinger wird in die aufklappbare Messvorrichtung eingelegt. Die übrigen Finger halten die Messvorrichtung fest, wobei sich an den Außenseiten des Gehäuses 1 ebenfalls Messeinheiten befinden, die hier für den rechten Zeigefinger und den rechten Daumen vorgesehen sind.

Mithilfe der äußeren und inneren Messeinheiten der Messvorrichtung sind verschiedene Arten von Messungen an den Fingern möglich:
- Optische Messungen: Messungen der Transmission und der diffusen Reflexion bei verschiedenen Wellenlängen am linken Zeigefinger.
- Elektrische Messungen: ECG-Messungen (zwischen linkem und rechtem Zeigefinger) und verschiedene Bioimpedanzmessungen (ebenfalls zwischen linkem und rechtem Zeigefinger, sowie rechtem Zeigefinger und rechtem Daumen).
- Temperaturmessung: Messungen der Fingertemperatur am rechten Zeigefinger.

Die Messung an anderen Fingern ist ebenso möglich. Beispielsweise könnte auch am Mittel- statt am Zeigefinger gemessen werden, oder die linke und rechte Hand vertauscht werden.

Um die von der Messvorrichtung generierten Daten auszulesen und weiterzuverarbeiten, verfügt die Erfindung über einen Mikrocontroller. In Abhängigkeit der zu messenden Parameter kann der Mikrocontroller dabei verschiedene Messprogramme ausführen, die sich hinsichtlich der benutzten Messeinheiten, sowie der Dauer und Reihenfolge der durchgeführten Messvorgänge unterscheiden. Die Dauer eines solchen Messprogramms liegt je nach Anwendungszweck und der zu bestimmenden Parameter des Anwenders zwischen wenigen Sekunden und mehreren Minuten.

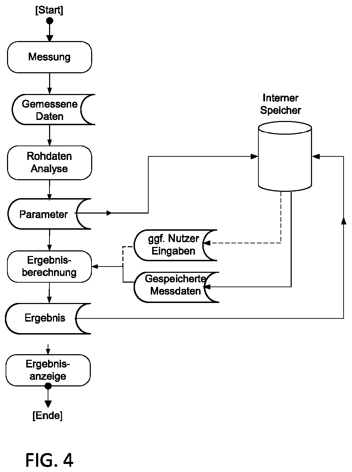

Wie der typische Ablauf eines Messprozesses, bestehend aus der Ausführung des Messprogrammes und der anschließenden Ergebnisberechnung, mit der erfindungsgemäßen Messvorrichtung aussehen kann, ist in den Figuren 3 und 4 dargestellt. Der typische Ablauf umfasst die folgenden Schritte:
- Der Nutzer nimmt die Messeinheit in die Hand und wählt über ein auf der Anzeige 2b der Messvorrichtung dargestelltes Menu mithilfe zweier Bedienelemente 2c (Knöpfe) eine Messung aus.
- Der Nutzer wird aufgefordert, den linken Zeigefinger in die Messvorrichtung einzulegen. Die Finger der rechten Hand werden auf den außen am Gehäuse 1 angeordneten Sensoren platziert und die Messvorrichtung wie in den Figuren 3 a, b dargestellt gehalten.
- Die Messvorrichtung erkennt über eine optische, elektrische und/oder Temperatur-Messung das Einlegen des Fingers und/oder den Kontakt der Finger mit den äußeren Sensoren.
- Es wird ein in der Mikrocontroller-Software hinterlegtes Messprogramm mit fester Dauer gestartet und einzelnen Messeinheiten der Messvorrichtung in vorgegebener Weise angesteuert und ausgelesen.
- Die gemessenen Daten werden analysiert und es werden für die einzelnen Messsignale spezifische Parameter berechnet.
- Basierend auf den gemessenen Parametern können weitergehende, gegebenenfalls statistische Analysen der Messdaten durchgeführt werden, die auch die Ergebnisse früherer Messungen berücksichtigen können.
- Das Ergebnis der Messung wird dem Nutzer angezeigt und die Resultate werden gespeichert. Bei dem angezeigten Ergebnis kann es sich entweder um einen direkt aus der Messung zu entnehmenden Parameter oder einen aus einer weitergehenden, evtl. statistischen Analyse bestimmten Parameter handeln.

Die Datenverarbeitung und Analyse kann entweder durch den geräteeigenen Mikrocontroller durchgeführt werden, oder die Daten werden an eine externe Datenverarbeitungseinheit übermittelt und dort weiterverarbeitet und ausgewertet. Die Datenübermittlung kann hier drahtgebunden oder auch drahtlos beispielsweise über Bluetooth oder Ähnliches realisiert sein.

Es ist somit auch möglich, die Benutzerschnittstelle zur Bedienung der Messvorrichtung auf der externen Datenverarbeitungseinheit, beispielsweise einem Smartphone oder Ähnlichem zu realisieren.

Unabhängig von der Gerätevariante ist die Messvorrichtung zur Erhöhung der elektrischen Sicherheit des Nutzers batterie- oder akkubetrieben.

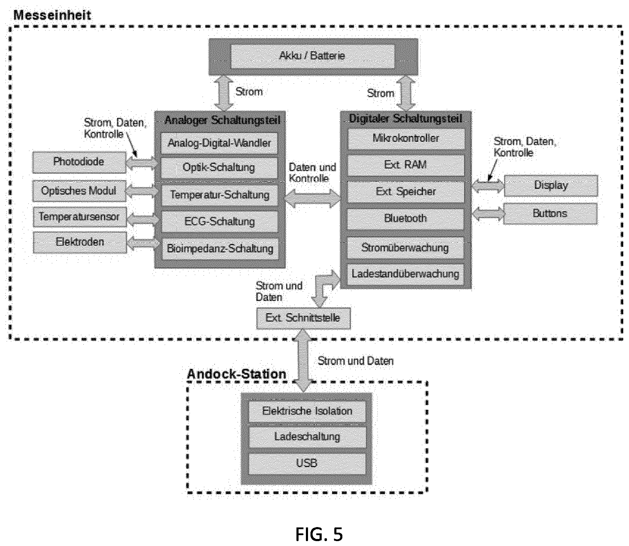

Zur Realisierung der Messfunktion, Analyse und Speicherung und gegebenenfalls Weiterleitung der Daten, sowie Benutzerinteraktion und Überwachung des Geräts verfügt die Erfindung über verschiedene Schaltungsteile. In einer möglichen Realisierung können die verschiedenen Schaltungsteile grob in einen analogen und einen digitalen Schaltungsteil getrennt werden. Das elektronische Konzept der Messvorrichtung ist für diesen Fall in Figur 5 dargestellt.

Der analoge Schaltungsteil enthält hier die für das Auslesen der Messeinheiten und die analoge Verarbeitung der Messsignale notwendige Elektronik (ECG-, Bioimpedanz-, Temperatur- und Optik-Schaltungen). Je nach Ausführungsform der Messvorrichtung können diese Schaltungsteile ein oder mehrere analoge Filterstufen beinhalten, müssen dies aber nicht. Die Daten der Messeinheiten werden für die weitere digitale Verarbeitung von einem oder mehreren Mehrkanal-ADCs (Analog-Digital-Converter) digitalisiert. Die aktiven Teile der Messeinheiten (Ansteuern der LEDs, Generierung des Wechselstroms für die Bioimpedanzmessungen) befinden sich ebenfalls im analogen Schaltungsteil.

Der digitale Schaltungsteil umfasst in der gezeigten Realisierung den für die Steuerung der Elektronik sowie die Verarbeitung der Messdaten notwendigen Mikrocontroller, zusammen mit zusätzlichem sowohl flüchtigen als auch persistenten Speicher. Außerdem befindet sich in diesem Schaltungsteil die Steuerung der Bedienelemente und der Anzeige. Zusätzlich können in diesem Schaltungsteil ein optionaler Bluetooth-Chip und weitere Elektronik zur Überwachung des Gerätezustands, einschließlich des Ladezustands der Batterie bzw. des Akkus, realisiert sein.

Bei Ausführungsformen der Erfindung, bei denen die Messdaten und/oder Ergebnisse an andere Geräte weitergeleitet werden, müssen jedoch nicht alle Schaltungsteile vorhanden sein: Beispielsweise ist denkbar, dass auf den persistenten Speicher außerhalb des Mikrocontrollers verzichtet wird, wenn die Speicherung von Messergebnissen auf einem anderen Gerät erfolgt.

Dahingegen muss die Schaltung bei Gerätevarianten ohne Andockstation gegebenenfalls um eine Ladeschaltung für den Akku und eine elektrische Schutzschaltung zur Erhöhung der elektrischen Sicherheit ergänzt werden. Bei Gerätevarianten mit Andockstation kann die Ladeschaltung für das Aufladen des Akkus vollständig in der Andockstation realisiert sein, wodurch das Volumen der Schaltung in der Messvorrichtung verringert werden kann. Kommunikation mit externen Geräten über kabelgebundene Schnittstellen wie USB erfolgt in diesem Fall ebenfalls ausschließlich über die Andockstation.

### Konzept der Mikrocontroller Software:

Die auf dem Mikrocontroller gespeicherte Software ermöglicht den Messvorgang, die Analyse der Messdaten, sowie die Interaktion der Messvorrichtung mit dem Nutzer sowie der Umgebung über entsprechende Schnittstellen und Protokolle (z.B. USB und Bluetooth).

Die möglichen Hauptaufgaben der Firmware sind:
- Durchführung eines Geräte-Selbsttests beim Anschalten der Messvorrichtung, sodass die Wahrscheinlichkeit von Fehlmessungen durch Hardwaredefekte verringert werden kann.
- Interaktion mit dem Nutzer über die Bedienelemente 2b und die Anzeige 2c (basierend auf einem graphischen Menü), auch zur Anzeige von Instruktionen für den Messvorgang.
- Durchführung verschiedener Arten von Messprogrammen, die die verschiedenen Messeinheiten auf verschiedene Arten kombinieren.
- Ansteuerung und Auslesen der Messelektronik.
- Analyse der Rohdaten und ggf. Berechnung weiterer Parameter mit statistischen Verfahren.
- Das Ergebnis kann sowohl als Zahlenwert als auch graphisch dargestellt werden. Ein Beispiel für Letzteres wäre die Darstellung eines farbigen Balkens, mit dem durch verschiedene Farben der Normbereich eines Parameters sowie von diesem Normbereich abweichende Werte dargestellt werden. In diesem Fall kann das Messergebnis dargestellt und für den Nutzer eingeordnet werden, indem ein Pfeil eingezeichnet wird, der auf eine bestimmte Position innerhalb des Balkens verweist.
- Speicherung und Laden von Nutzereingaben, Konfigurationsdateien und Messdaten.
- Anzeige früherer Messwerte.
- Kommunikation mit der Andockstation sowie mit der Umgebung über die externen Schnittstellen, die gegebenenfalls über die Andockstation realisiert sind (z.B. USB-Schnittstelle).
- Weitere Hilfs-Funktionen wie Sprachauswahl, Anzeige von Geräteinformationen usw.
- Es müssen nicht alle aufgelisteten Funktionen (z.B. die Analyse der Rohdaten) in der Mikrocontroller-Software realisiert sein. Teilschritte können auch auf ein anderes Gerät ausgelagert sein.
- Auf das prinzipielle Vorgehen bei der Ausführung von Messprogrammen sowie der Analyse der resultierenden Daten wird im Folgenden näher eingegangen.

### Ausführung vorgegebener Messprogramme:

Die erfindungsgemäße Messvorrichtung erlaubt die Ausführung verschiedener, in der Mikrocontroller-Software definierter Messprogramme. Diese Messprogramme können sich hinsichtlich der Dauer und der Art der durchgeführten Teilmessungen bzw. der verwendeten Sensorik unterscheiden. Welches Messprogramm verwendet wird, hängt von den jeweiligen Zielparametern ab. Beispiele für mögliche Zielparameter und dazugehörige Messprogramme sind wie folgt:
- Berechnung der Herzrate des Nutzers oder anderer ECG-Parameter aus einer ECG-Messung.
- Bestimmung eines Indikators für die Pulswellengeschwindigkeit des Nutzers durch simultane Messung eines Photopletyhsmograms und eines ECG.
- Bestimmung der arteriellen Sauerstoffsättigung durch optische Messungen am Finger bei verschiedenen Wellenlängen.
- Messungen der Bioimpedanz entlang der durch die Messelektroden 7 vorgegebenen Messstrecken bei einer bestimmten Frequenz (z.B. 50 kHz). Es sind auch Messungen mit mehreren Frequenzen, einschließlich des Durchfahrens eines bestimmten Frequenzbereichs, denkbar.
- Messung der Fingertemperatur des Nutzers über den mit dem Finger in Kontakt stehenden Temperatursensor 8 der Messvorrichtung.

Die vorgenannten, beispielhaft dargestellten Messprogramme können auch miteinander kombiniert werden, sodass mehrere Zielparameter innerhalb desselben Messprogramms bestimmt werden können.

Zu beachten ist, dass gewisse Zielparameter mit mehreren Messeinheiten bestimmt werden können, sodass die Messergebnisse der Einzelmessungen miteinander verglichen und auf Plausibilität geprüft werden können. Insbesondere kann die Bestimmung je nach verwendeten Messeinheiten auch simultan erfolgen. Dadurch kann die Aussagekraft der Ergebnisse erhöht werden. Beispiele für solche Mehrfachbestimmungen sind wie folgt:
- Bestimmung der Herzrate aus der ECG-Messung und aus einer photoplethysmographischen Messung mit den optischen Messeinheiten.
- Bestimmung plethysmographischer Parameter aus den optischen Messungen (Photoplethysmographie) sowie aus den Impedanzmessungen (Impedanzplethysmographie).
- Bestimmung oxymetrischer Parameter aus den optischen Messungen sowie aus den thermischen Messungen.

Für den Endnutzer kann die Mikrocontroller-Software so konfiguriert werden, dass entweder ein vorgegebenes Messprogramm ausgeführt wird oder zwischen verschiedenen Messprogrammen ausgewählt werden kann.

### Analyse der gemessenen Daten:

Grundsätzlich kann die Analyse der Messdaten konzeptionell in zwei Hauptschritte unterteilt werden:
1. Analyse der Messsignale und Ableitung spezifischer Parameter aus den Messsignalen.
2. Anwendung weiterer, ggf. statistischer Verfahren zur Berechnung weiterer Parameter, die der direkten Messung nicht zugänglich sind.

Je nach Anwendung müssen nicht beide Schritte der Analyse realisiert sein. Soll beispielsweise nur die Herzrate des Nutzers gemessen und angezeigt werden, dann reicht es, diese aus den Messsignalen direkt abzuleiten. Eine weitere statistische Analyse ist nicht erforderlich.

Für die Analyse der Messsignale können in der Mikrocontroller-Software verschiedene an die Charakteristik des jeweiligen Messsignales sowie für die Berechnung der Zielparameter spezifisch angepasste Funktionen ausgeführt werden. Zu solchen Funktionen zählen:
- Vorverarbeitung der Messsignale (z.B. Basislinien-Korrektur, Rauschunterdrückung)
- Berechnung statistischer Kenngrößen der Messsignale.
- Berechnung charakteristischer Punkte in den Messsignalen.
- Bewertung der Signalformen bei Signalen mit charakteristischem zeitlichem Verlauf (z.B. ECG).
- Kombination der Informationen verschiedener Messsignale (z.B. Bestimmung der arteriellen Sauerstoffsättigung und des Sauerstoffverbrauches in den arteriellen/venösen Geweben anhand der unterschiedlichen Absorptionscharakteristiken des Fingers bei verschiedenen Wellenlängen).

Nicht alle dieser Schritte müssen je nach Anwendungsfall realisiert sein.

Die von der Messvorrichtung erhaltenen Parameter werden auch auf verschiedene Weise standardisiert und entsprechend sowohl der physiologischen als auch physikalischen Kalibration gewichtet. Die Beziehung zwischen den Parametern und z.B. dem Blutzuckerspiegel kann mittels mathematischer Modelle hergestellt und mithilfe von Biostatistik bestätigt werden. Hierzu können die Parameter der Einzelsignale sowie mögliche kombinierte Parameter für ein ausgewähltes statistisches Verfahren verwendet werden.

Die Daten können über Vorrichtungen zur Datenweiterleitung auch in einer externen Datenbank gespeichert sein. Das mithilfe des statistischen Verfahrens berechnete Ergebnis kann dem Nutzer dann angezeigt und gegebenenfalls im internen Speicher der Messvorrichtung oder einer Datenbank hinterlegt werden.

### Varianten der Messvorrichtung:

Die in den vorherigen Abschnitten dargestellten Schritte, einschließlich der Bestimmung des Blutzuckerspiegels, können direkt auf der Messvorrichtung stattfinden (Standalone-Variante). Alternativ kann die Analyse der Daten, z.B. wenn diese zu rechenaufwendig für die Messvorrichtung ist, auch auf ein weiteres Gerät oder einen Server ausgelagert werden (Remote-Variante). In diesem Fall finden einzelne oder alle Prozessschritte, die nach der Aufnahme der Messdaten erfolgen, einschließlich der Speicherung der Daten, auf einem anderen Gerät, z.B. einem Server des Herstellers oder einer anderen vertraglich gebundenen Organisation, statt.

Die Messvorrichtung wird über drahtlose Kommunikation, beispielsweise mittels Bluetooth, mit einem weiteren Gerät wie einem PC oder Mobiltelefon verbunden. Auf dem weiteren Gerät wird eine spezielle Applikation ausgeführt, die mit der Messvorrichtung kommuniziert. Eine wesentliche Aufgabe dieser Applikation besteht in diesem Anwendungsfall darin, die Messdaten über eine Internetverbindung an einen Server weiterzuleiten. Dies kann in Form eines Streamings während der Messung oder durch Versenden der vollständigen Messdaten nach Abschluss der Messung geschehen.

Die Messdaten werden anschließend auf dem Server analysiert. Das auf dem Server berechnete Ergebnis der Messung kann dann auf dem externen Gerät oder der Messvorrichtung angezeigt werden.

Die auf dem externen Gerät laufende Applikation kann darüber hinaus die Funktionalität der Messvorrichtung erweitern, indem beispielsweise eine graphische Anzeige der Historie der Messwerte oder ein Export der Messergebnisse für weitere Verwendung implementiert wird.

### Erweiterungsmöglichkeiten der Erfindung:

Die erfindungsgemäße Messvorrichtung kann auf vielfältige Weise erweitert werden, ohne dass sich der Kern der Erfindung ändert. Zu den bereits oben erwähnten, generellen Erweiterungs- und Änderungsmöglichkeiten gehören insbesondere:
- Die Anzahl, Art und Position der Sensoren der verschiedenen Messeinheiten.
- Die Form und Größe des Gehäuses 1.
- Die durchgeführten Messprogramme und die aus den Messdaten berechneten Zielparameter.
- Die Realisierung von Standalone- und Remote-Varianten der Messvorrichtung, die z.B. drahtlose Kommunikationsmöglichkeiten wie Bluetooth nutzen.
- Die Verwendung der Messvorrichtung mit und ohne Andockstation, sowie mit fest verbautem oder austauschbarem Akku. Bei Varianten ohne Andockstation können externe Schnittstellen wie USB direkt in das Gerät integriert sein.

Zusätzliche, konkrete Erweiterungsmöglichkeiten werden im Folgenden beschrieben. Die Erweiterungsmöglichkeiten sind dabei thematisch gruppiert.

### Erweiterung der ECG- und Bioimpedanz-Messungen:

Es können zusätzliche Elektroden für weitere Bioimpedanz-Messungen zur Messvorrichtung hinzugefügt oder die vorhandenen Elektroden für andere Messungen genutzt werden, z. B.:
- Messung der Impedanz zwischen linkem Zeigefinger und rechtem Daumen mit den vorhandenen Elektroden.
- Hinzufügen zweier zusätzlicher Elektroden in der inneren Fingermulde oder an den Außenseiten der Messvorrichtung, sodass die Impedanz an einem einzelnen Finger gemessen werden kann (lokale Messung).
- Zwei zusätzliche Elektroden an der Außenseite, sodass die Impedanz zwischen den beiden Daumen gemessen werden kann.
- Externe Anschlüsse für weitere (Klebe-)Elektroden mit Kabeln, sodass die Impedanz auch an anderen Körperstellen als an den Fingern gemessen werden kann (z.B. am Arm).
- Zusätzliche Elektroden an der Rückseite des Geräts, sodass die Messvorrichtung an den Körper gepresst und die Bioimpedanz zwischen eingelegtem Finger und der entsprechenden Körperstelle gemessen werden kann.

Es können zusätzliche Elektroden hinzugefügt oder die vorhandenen Elektroden anders genutzt werden, um alternative ECG-Messung durchzuführen:
- Mit einer weiteren Elektrode an der Außenseite der Messvorrichtung könnte die ECG-Messung auch an den Daumen durchgeführt werden.
- Es können mehrere Elektroden für die ECG-Messung zusammengeschaltet werden, um die effektiv genutzte Elektrodenfläche zu vergrößern (z.B. Zusammenschalten der beiden inneren Elektroden am linken Zeigefinger und Zusammenschalten der beiden äußeren Elektroden am rechten Zeigefinger).
- Es können Anschlüsse für weitere externe Elektroden hinzugefügt werden, sodass Mehrkanal-ECG-Messungen durchgeführt werden können oder ein "Right Leg Drive" verwendet werden kann, um Gleichtaktstörungen zu reduzieren.
- Es können weitere Elektroden hinzugefügt werden, sodass die für die ECG-Messung und die Bioimpedanz-Messung genutzten Elektroden schaltungstechnisch vollständig getrennt sind.

Die Distanz zwischen den stromeinspeisenden und den spannungsmessenden Elektroden 7 der Bioimpedanz kann variiert werden.

Die Geometrie der Elektroden kann verändert werden:
- Die Elektroden können verkleinert oder vergrößert werden.
- Die Form der Elektroden 7 kann verändert werden (z.B. Verwendung kreisförmiger Elektroden).
- Zur effektiven Nutzung des Platzes in der Kammer 9 kann eine der Elektroden 7 in der Kammer 9 so ausgeformt sein, dass sich das optische Modul 11 in einer Aussparung innerhalb der Elektrode befindet.

Das Material der Elektroden kann geändert werden (z.B. Verwendung spezieller Stahlsorten oder eines vollkommen anderen Materials).

Die Oberfläche der Elektroden kann verändert werden (z.B. Verwendung glatter oder angerauter Elektroden).

Zur Verbesserung der ECG- oder Bioimpedanzmessungen kann eine Flüssigkeit (auch Wasser) oder eine Form von Kontaktgel auf die Elektroden bzw. auf die Finger aufgetragen werden.

Statt fest verbauter Elektroden können auch austauschbare Elektroden verwendet werden. Beispielsweise können in diesem Fall Ag/AgCI-Elektroden verwendet werden, die erst kurz vor der Messung in das Gerät eingelegt und nach der Messung wieder entfernt werden.

Die Bioimpedanzmessung kann bipolar, tripolar, oder tetrapolar durchgeführt werden. Es ist auch eine matrixförmige Anordnung von Elektroden möglich, in der mit verschiedenen Kombinationen von Elektroden gemessen werden kann.

Die Bioimpedanzmessungen können sowohl mit konstantem Strom als auch konstanter Spannung durchgeführt werden.

Um Probleme bei der Bioimpedanzmessung zu erkennen (z.B. durch zu hohe Übergangswiderstände am Finger), kann über Erweiterungen der Bioimpedanzschaltung der tatsächlich bei der Bioimpedanzmessung fließende Strom gemessen werden. Außerdem kann der zeitliche Verlauf des Stroms (z.B. Sinusform) geprüft werden.

### Erweiterung der optischen Messeinheiten:

- Die Anzahl der optischen Sensoren kann geändert werden; beispielsweise könnte auf den optische Sensor im oberen Teil der Kammer verzichtet werden oder ein weiterer optischer Sensor in einem gewissen Abstand zum vorhandenen Sensor hinzugefügt werden. Mit einem weiteren Sensor könnte man Laufzeitdifferenzen bestimmen oder Gewebeeigenschaften räumlich auflösen.
- Es wäre ebenfalls denkbar, ein Array oder eine matrixförmige Anordnung optischer Sensoren statt einzelner optischer Sensoren im oberen Teil der Kammer zu verwenden, sodass beispielsweise optische Gewebeeigenschaften räumlich aufgelöst werden können.
- Es könnte ein anderes optisches Modul im unteren Teil der inneren Fingerauflage verwendet werden, das beispielsweise Lichtquellen mit Wellenlängen enthält, die für eine spezielle Anwendung optimiert sind.
- Ebenso könnte ein Array oder eine matrixförmige Anordnung von optischen Sensoren in das optische Modul der unteren Fingerauflage integriert sein.
- Die Eigenschaften der optischen Sensoren können je nach Anwendungszweck angepasst werden; beispielsweise könnten Sensoren mit unterschiedlichen aktiven Flächen oder unterschiedlichen Empfindlichkeiten in bestimmten Wellenlängenbereichen verwendet werden.
- Der Abstand zwischen dem optischen Modul und dem gegenüberliegenden weiteren optischen Sensor kann variiert werden, sodass der Lichtweg durch das Gewebe länger oder kürzer wird bzw. das Licht andere Gewebeanteile durchquert hat, bevor es detektiert wird.
- Die Messvorrichtung verfügt über mehrere optische Sensoren, die entlang des Fingers platziert sind. Da der Abstand der Sensoren bekannt ist, kann man aus der Zeitdifferenz, mit der die unterschiedlichen optischen Sensoren die Änderung der Gewebeabsorption aufgrund der Pulsation des arteriellen Blutes detektieren, einen Indikator für die Pulswellengeschwindigkeit ohne zusätzliche ECG-Messung berechnen. Es wäre in diesem Fall günstig, den Abstand der optischen Sensoren im Messsystem möglichst groß zu wählen, damit die zu messende Zeitdifferenz ebenfalls möglichst groß wird.
- Der Abstand zwischen den Lichtquellen und optischen Sensoren kann geändert werden, sodass sich die Eindringtiefe des Lichts in das Gewebe ändert.
- Die Intensität der Lichtquellen kann variiert werden und an die Charakteristik des Nutzers, z.B. je nach Fingerdicke, über einen Verstärkungsfaktor angepasst werden.
- Zusätzlich zu den Fingermessungen können auch Leermessungen (Messungen der Intensität der Lichtquellen ohne eingelegten Finger) in der Messvorrichtung durchgeführt werden. Enthält das optische Modul jedoch Sensorik zur Bestimmung der Intensität der Lichtquellen, dann können solche Messungen entfallen.
- Im Falle einer Leermessung zur Intensitätsnormierung kann die Leermessung bei einer anderen Verstärkung als die Fingermessung erfolgen, sodass Sättigungen der optischen Sensoren bei der Leermessung vermieden werden.
- Die Lichtquellen können mit Multiplexing- oder Modulationsverfahren betrieben werden statt diese sequentiell zu aktivieren. Das resultierende Signal der optischen Sensoren muss dann dementsprechend mit De-Multiplexing oder De-Modulation in die Anteile der verschiedenen Lichtquellen zerlegt werden.
- Bei den Lichtquellen im Optikmodul kann es sich neben LEDs auch um andere Lichtquellen, z.B. Diodenlaser oder Quantenpunkt-Leuchtdioden (QLEDs), handeln.
- Auf Multiplexing oder Modulation beim Betrieb mehrerer Lichtquellen könnte verzichtet werden, wenn die optischen Sensoren in Form eines miniaturisierten Spektrometers realisiert wären.

### Erweiterung der Temperaturmessungen:

- Die Anzahl der Temperatursensoren kann variiert werden. Beispielsweise können separate Temperatursensoren für die Bestimmung der Gehäusetemperatur, der Temperatur der Elektronik, und der Temperatur des Fingers verwendet werden.
- Je nach Art und Positionierung der Temperatursensoren können verschiedene Arten von Temperaturmessungen durchgeführt werden, z.B. mit und ohne direkten Kontakt zum Sensor: Bei direktem Kontakt zwischen Sensor und Finger wird die Temperatur durch Wärmeleitung bestimmt. Es ist aber auch denkbar, dass beispielsweise die Wärmestrahlung des Fingers gemessen wird bzw. die Körpertemperatur durch eine kontaktlose Messung bestimmt wird.
- Soll die Temperatur des Fingers anhand der von diesem emittierten Wärmestrahlung bestimmt werden, dann kann ein strahlungssensitiver Temperatursensor so in eine Vertiefung des Gehäuses integriert werden, dass zwar Wärmestrahlung des Fingers den Temperatursensor erreichen kann, aber kein direkter Kontakt mit dem Finger besteht. In diesem Fall kann zur Verbesserung der Genauigkeit der auf der Strahlungsmessung basierenden Temperaturbestimmung ein zweiter baugleicher Temperatursensor verwendet werden, der sowohl gegen direkten Kontakt mit dem Finger als auch gegen Wärmestrahlung des Fingers abgeschirmt ist. Die Messung des zweiten Temperatursensors kann dann als Referenzmessung für den ersten Temperatursensor dienen. Die Abschirmung des zweiten Temperatursensors kann z.B. durch Integration des Temperatursensors in einen geschlossenen Hohlraum innerhalb der Gehäusewand geschehen.

### Erweiterung der Gerätemechanik:

- Der Federmechanismus kann derart geändert werden, dass die Feder austauschbar oder hinsichtlich der Federstärke einstellbar ist, damit für Nutzer mit unterschiedlich dicken Fingern ein gleicher Druck realisiert werden kann.
- Statt eine Rückwand am Ende der Fingermulde zur Positionierung des Fingers zu verwenden, kann auch eine tastbare Erhebung in die Auflagefläche der Kammer integriert werden, über die dem Nutzer die korrekte Fingerposition signalisiert wird.
- Die Messvorrichtung kann so konzipiert werden, dass diese wasserdicht und staubdicht ist. Außerdem kann das Gehäuse so modifiziert werden, dass dieses Stürze aus großer Höhe übersteht.
- Die Form des Gehäuses kann variiert werden; beispielsweise ist auch ein ovales Gehäuse denkbar. Exakte Länge, Breite, Höhe, sowie Farbe, verwendetes Gehäusematerial oder Oberflächenstruktur des Gehäusematerials ändern die Erfindung ebenfalls nicht.
- Die Geometrie der Fingerauflage, beispielsweise der Krümmungsradius oder die Länge der Fingerauflage, kann verändert werden, damit das Gerät von unterschiedlichen Nutzergruppen, z.B. Kindern und Erwachsenen, gleichermaßen verwendet werden kann.

### Verbesserung der Handhabung der Messvorrichtung:

- Um die Handhabung der Messvorrichtung zu vereinfachen und gleichzeitig eine reproduzierbare Position der Finger auf den äußeren Sensoren zu ermöglichen, können an den Außenseiten der Messvorrichtung Stopper angebracht werden. Alternativ kann man die äußeren Sensoren auch in flache Mulden integrieren, die die Fingerposition vorgeben.

### Erweiterungen der Messprogramme:

- Eine Weiterbildung der Erfindung sieht vor, dass das für die Aufnahme der Messdaten genutzte Messprogramm keine feste Dauer hat, sondern sich dynamisch an die Qualität der Messdaten bzw. den Analysezweck anpasst. Beispielsweise ist denkbar, dass für eine ECG-Messung solange gemessen wird, bis eine bestimmte Anzahl ECG-Pulse gemessen wurde statt eine feste Messdauer vorzugeben. Genauso ist denkbar, dass bei verschiedenen Messungen das Signal-zu-Rausch-Verhältnis des Messsignals berücksichtigt wird und dementsprechend bei Nutzern mit schlechtem Signal-zu-Rausch-Verhältnis länger gemessen wird als bei Nutzern mit gutem Signal-zu-Rausch-Verhältnis.
- Für bestimmte Anwendungsfälle kann es günstig sein, wenn die Messvorrichtung zwischen Trainingsmessungen (keine Ergebnisanzeige) und Testmessungen (mit Ergebnisanzeige) unterscheidet. Die Trainingsmessungen können beispielsweise genutzt werden, damit sich der Nutzer mit der Handhabung des Geräts vertraut macht.

### Erweiterungen der Datenanalyse:

- Eine Weiterbildung der Erfindung sieht vor, in einem oder beiden der oben genannten konzeptionellen Analyseschritte (bestehend aus der Parameterextraktion aus den Messdaten sowie der Anwendung statistischer Methoden für die modellbasierte Berechnung weiterer Parameter) Verfahren des maschinellen Lernens zu verwenden.
- Dabei können für unterschiedliche Teilschritte verschiedene maschinelle Lernverfahren eingesetzt und auch kombiniert werden. Zu den Verfahren, die hierzu eingesetzt werden können, zählen insbesondere neuronale Netze, Support Vector Machines und Decision Trees einschließlich Random Forests oder davon abgeleitete Verfahren.
- Die maschinellen Lernverfahren können beispielsweise die Berechnung der Parameter aus den Messsignalen unterstützen oder z.B. auch die Anwendung von Methoden der klassischen Signalverarbeitung teilweise oder vollständig ersetzen. Genauso können die maschinellen Lernverfahren eingesetzt werden, um anhand von Trainingsdaten Modelle für die Berechnung weiterer Parameter, die der Messung nicht direkt zugänglich sind, zu generieren.
- Ebenso können mithilfe maschineller Lernverfahren auch mehrere Analyseschritte simultan ausgeführt werden. Dies beinhaltet insbesondere, dass die Schritte der Parameterextraktion aus den Rohdaten sowie der modellbasierten Berechnung weiterer Parameter kombiniert werden können. Hierzu bietet sich die Verwendung tiefer neuronale Netze (sogenanntes Deep Learning) an, da tiefe neuronale den Prozess der Parameterextraktion automatisieren können, sodass diese nicht mehr explizit definiert und berechnet werden müssen.
- Je nach Komplexität der verwendeten Modelle können diese entweder direkt in die Software der Messvorrichtung integriert sein und von der Software des Mikrocontrollers ausgewertet werden oder auf einem anderen Gerät ausgewertet werden, zu dem die Messdaten oder Parameter über die Mittel zur Datenweiterleitung übertragen werden.

### Erweiterte Nutzung der Daten der Messvorrichtung:

- Statt einzelne Parameter als Ergebnis auf der Messvorrichtung anzuzeigen, können Messdaten auch auf ein anderes Gerät, z.B. ein Personal Computer, übertragen werden, sodass die gemessenen Signale (z.B. ECG) direkt z.B. von medizinischem Personal mithilfe einer entsprechenden Applikation betrachtet und bewertet werden können. Ebenso können die Historien verschiedener physiologischer Parameter übertragen werden, um z.B. die Entwicklung des Gesundheitszustands eines Anwenders über einen längeren Zeitraum durch medizinisches Personal bewerten zu lassen.
- Es können dem Nutzer verschiedene Messprogramme angeboten werden, mit deren Hilfe je nach Bedarf und Interesse verschiedene Parameter (z.B. Herzrate, Sauerstoffverbrauch, Blutdruck oder Blutzuckerspiegel) gemessen werden können. Es können separate Historien für die gemessenen Parameter angelegt und dem Nutzer angezeigt werden.
- Werden die mit der Messvorrichtung generierten Ergebnisse (z.B. Herzrate oder Blutzucker) auf ein anderes Gerät, z.B. ein Personal Computer oder ein Smartphone, übertragen werden, dann kann dieses Gerät zusätzlich mit einer Datenbank verbunden sein, in der der Nutzer Angaben zu seinen Lebensgewohnheiten (Art und Häufigkeit von Mahlzeiten, Bewegung etc.) hinterlegt. Durch Verknüpfung von Messergebnissen und Angaben zu Lebensgewohnheiten können die Auswirkungen der Lebensgewohnheiten auf den Nutzer, z.B. die Art der Ernährung auf dessen gemessenen Blutzucker, überwacht werden. Umgekehrt können die zusätzlich hinterlegten Informationen auch zur Verbesserung der modellbasierten Berechnung von Parametern verwendet werden.
- Bei nur für bestimmte Personengruppen anwendbaren Zusammenhängen zwischen Messdaten und Zielparametern kann die Zuordnung eines Nutzers zu einer solchen Personengruppe auch durch Anwendung eines statistischen Verfahrens (z.B. Clustering) anhand der gemessenen Parameter erfolgen.
- Basierend auf einer Historie gemessener Parameter (z.B. nach einer Trainingsphase) kann der Nutzer anhand seiner Messwerte mithilfe statistischer Verfahren wiedererkannt bzw. identifiziert werden. Dadurch kann die Messvorrichtung personalisiert verwendet werden. Insbesondere bei nutzerspezifischer Konfiguration der Messvorrichtung kann damit auch verhindert werden, dass ein Nutzer versehentlich eine falsche Konfiguration verwendet.
- Für bestimmte Anwendungsfälle ist ebenfalls denkbar, dass ein Zusammenhang zwischen den gemessenen Parametern und einem Zielparameter, beispielsweise Blutzucker, für bestimmte Nutzer in Zusammenarbeit mit medizinisch geschultem Personal entwickelt wird und dieser Zusammenhang dann in der Messvorrichtung für diesen speziellen Nutzer in Form einer Konfigurationsdatei hinterlegt wird.

### Verbesserung der Benutzerfreundlichkeit:

- Die Anzeige der Messvorrichtung kann entspiegelt werden, sodass die Anzeige der Messvorrichtung auch bei starkem Lichteinfall gut abgelesen werden kann.
- Es können Status-LEDs in das Gehäuse der Messvorrichtung integriert werden, die beispielsweise den Ladezustand der Batterie bzw. des Akkus anzeigen.
- Die Orientierung der Anzeige der Messvorrichtung kann geändert werden (z.B. Einbau der Anzeige längs statt quer). Außerdem könnte man mithilfe eines Gyroskopsensors Änderungen der Orientierung des Geräts feststellen, sodass die Orientierung der Anzeige durch die Software automatisch geändert wird.
- Die Messvorrichtung kann so erweitert werden, dass akustische Signale, beispielsweise für das Ende der Messung, generiert werden können.
- Es ist ebenfalls denkbar, dass die Messvorrichtung um eine Sprachausgabe erweitert wird, sodass das Ergebnis oder die Instruktion dem Nutzer per Sprachausgabe mitgeteilt werden können. Dadurch würde sich die Nutzbarkeit des Geräts für Menschen mit eingeschränkter Sehfähigkeit verbessern.
- Zu Informationszwecken können dem Nutzer während der Messung auf der integrierten Anzeige der Messvorrichtung Messdaten angezeigt werden.

### Erweiterung der Nutzung der drahtlosen Kommunikation, z.B. Bluetooth:

- Wenn die Messvorrichtung über drahtlose Kommunikation mit einem anderen Gerät, z.B. einem Smartphone verbunden wird, kann die Anzeige des Smartphones als ergänzende Anzeige für die Messvorrichtung oder als vollständiger Ersatz für die Anzeige der Messvorrichtung verwendet werden. Da die Anzeige eines Smartphones typischerweise deutlich größer als die Anzeige der Messvorrichtung ist, können in diesem Fall z.B. detailliertere Messstatistiken angezeigt oder das Ergebnis mit deutlich größerer Schrift angezeigt werden, wobei Letzteres insbesondere für Menschen mit eingeschränkter Sehfähigkeit hilfreich ist. Das Smartphone oder ein anderes externes Gerät kann auch vollständig als Nutzer-Schnittstelle für die Messvorrichtung verwendet werden, sodass die Bedienelemente, die in die Messvorrichtung integriert sind, reduziert werden können.
- Die Software der Messvorrichtung und deren Kommunikationsprotokolle können so angepasst werden, dass diese mit standardisierten Kommunikationsprotokollen, z.B. im Netzwerk eines Krankenhauses, kompatibel sind und die Ergebnisse der Messvorrichtung direkt in dieses Netzwerk übertragen werden können.
- Softwareaktualisierungen oder Aktualisierungen z.B. von Konfigurationsdaten der Messvorrichtung können auch über drahtlose Kommunikation erfolgen, indem diese beispielsweise von einem Smartphone an die Messvorrichtung übertragen werden.
- Wenn die Andockstation über einen eigenen Speicher verfügt, kann die Fähigkeit der Messvorrichtung zur drahtlosen Kommunikation auch als Datenübertragungsweg zwischen Andockstation und Umgebung verwendet werden. In diesem Fall können über die drahtlose Verbindung auch Konfigurations- oder Kalibrationsdaten an die Andockstation übertragen und in deren Speicher abgelegt werden.
- Es ist denkbar, dass sich die Messvorrichtung vor der Durchführung von Messungen, z.B. aus Sicherheitsgründen, erst über drahtlose Kommunikation (ggf. unter Nutzung der Internetverbindung beispielsweise eines Smartphones) bei einem vom Hersteller betriebenen Server authentisieren muss. Die Authentifizierung könnte z.B. auf dem Austausch spezieller kryptographisch signierter Schlüssel basieren.
- Das beschriebene Konzept mit Authentifizierung der Messvorrichtung bei einem Server könnte auch genutzt werden, um ein Bezahlsystem für das Messsystem zu realisieren.

### Diverse Erweiterungsmöglichkeiten:

- Für bestimmte Anwendungsfälle der Messvorrichtung, z.B. in einem Krankenhaus, ist denkbar, die in der Messvorrichtung befindliche Elektronik derart zu reduzieren, dass sich nur der für das Auslesen der Sensordaten und die anschließende Digitalisierung der Daten notwendige Teil der Elektronik in der Messvorrichtung befindet. Die gemessenen Daten könnten dann per Kabel oder drahtlos an eine Steuer- und Auswerteeinheit übertragen werden, die die Daten analysiert und die Daten oder aus diesen Daten berechnete Ergebnisse auf einem Monitor anzeigt.
- Das Geräte- und Multisensorkonzept kann auf eine Uhr bzw. eine Smartwatch oder einen Ring übertragen werden, den bzw. die der Nutzer permanent tragen kann. Dadurch lassen sich beispielsweise Messungen des Blutzuckers einfach in den Tagesverlauf des Nutzers integrieren, ohne dass ein zusätzliches Gerät transportiert werden muss.
- Der Akku der Messvorrichtung kann fest verbaut oder austauschbar sein. In letzterem Fall könnte man den Akku auch mit einem externen Ladegerät statt über die Andockstation laden.
- Die Andockstation kann über eine oder mehrere der folgenden Funktionen verfügen: Aufladen des Akkus der Messvorrichtung über einen externen Anschluss der Andockstation (z.B. USB).
- Bereitstellung weiterer kabelgebundener Schnittstellen, z.B. USB. Über diese Schnittstellen kann beispielsweise neben dem Transfer von Messdaten auch die Durchführung von Software-Updates realisiert werden oder die Aktualisierung von Konfigurationsdaten erfolgen.
- Elektrischer Schutz des Nutzers durch elektrische Isolation (galvanische Trennung) der Strom- und Datenleitungen vom Netzstrom.
- Zusätzlicher Schutz des Anwenders durch Implementierung eines Mechanismus, der verhindert, dass die Elektroden der Messvorrichtung berührt werden können, während die Messvorrichtung in die Andockstation eingelegt ist und geladen wird.
- Bereitstellung eines Transportbehältnisses, in dem die Messvorrichtung sicher transportiert werden kann.
- Prüfung oder Kalibration der Messvorrichtung, wenn in die Andockstation Mittel zur Prüfung oder Kalibration einer oder mehrerer Sensoreinheiten der Messvorrichtung integriert sind.

Ein Vorteil der Geräte-Variante mit Andockstation ist beispielsweise, dass Ladeschaltung und elektrische Schutzschaltung nicht in die Messvorrichtung integriert sein müssen, sodass das Volumen der elektrischen Schaltung in der Messvorrichtung verkleinert werden kann.

### Bezugszeichenliste:

- 1: Gehäuse
- 2: Oberschale
- 2a: Steg auf der Oberschale
- 2b: Anzeige
- 2c: Bedienelemente
- 3: Unterschale
- 3a: Vertiefung in der Unterschale
- 4: Klappmechanismus
- 5: Konnektor
- 6: Metallkontakte
- 7: Elektroden
- 8: Temperatursensor
- 9: Kammer
- 9a: Weiches Material
- 10: Wand
- 11: optisches Modul
- 12: Lichtquelle
- 13: weiterer optischer Sensor

## Patentansprüche

1. Multifunktionale Messvorrichtung aufweisend ein Gehäuse (1) mit einer Oberschale (2) und einer Unterschale (3), welche mittels eines Klappmechanismus (4) gegeneinander bewegbar sind und die miteinander korrespondierende Mulden aufweisen, wobei die Mulden eine von außen zugängliche Kammer (9) zur Aufnahme eines menschlichen Fingers bilden, wobei in der Kammer (9) eine optische Messeinheit mit einem optischen Modul (11), welches mindestens eine Lichtquelle (12) und mindestens einen Sensor aufweist, angeordnet ist und wobei im oder am Gehäuse Mittel zur Datenauswertung und/oder Datenweiterleitung integriert sind, wobei mindestens eine elektrische Messeinheit vorgesehen ist, mit mindestens zwei Messelektroden (7) in der Kammer (9),
**dadurch gekennzeichnet, dass** sich an zwei Seiten der Unterschale (3), an zwei Außenseiten der Messvorrichtung, jeweils zwei Messelektroden (7) für Bioimpedanz- und ECG-Messungen und an einer der Seiten zusätzlich ein Temperatursensor (8) befinden.

2. Multifunktionale Messvorrichtung nach Anspruch 1, **dadurch gekennzeichnet, dass** mindestens eine Temperaturmesseinheit im und/oder am Gehäuse (1) angeordnet ist.

3. Multifunktionale Messvorrichtung nach Anspruch 1 oder 2, **dadurch gekennzeichnet, dass** mindestens ein weiterer optischer Sensor (13) und/oder eine weitere Lichtquelle in der Kammer (9) gegenüber dem optischen Modul (10) angeordnet ist.

4. Multifunktionale Messvorrichtung nach einem der vorgenannten Ansprüche, **dadurch gekennzeichnet, dass** der Klappmechanismus mit einem Rückstellmechanismus versehen ist.

5. Multifunktionale Messvorrichtung nach einem der vorgenannten Ansprüche, **dadurch gekennzeichnet, dass** zur Datenauswertung ein Mikrocontroller im Gehäuse (1) angeordnet ist.

6. Multifunktionale Messvorrichtung nach einem der vorgenannten Ansprüche, **dadurch gekennzeichnet, dass** die Mittel zur Datenweiterleitung über eine Drahtlosschnittstelle verfügen.

7. Multifunktionale Messvorrichtung nach einem der vorgenannten Ansprüche, **dadurch gekennzeichnet, dass** Vorrichtungen zur Positionierung der einzelnen Finger vorgesehen sind, derart dass die Finger sich beim Messvorgang immer in der gleichen Position befinden.

8. Multifunktionale Messvorrichtung nach einem der vorgenannten Ansprüche, **dadurch gekennzeichnet, dass** ein Beschleunigungssensor integriert ist.

9. Multifunktionale Messvorrichtung nach einem der vorgenannten Ansprüche, **dadurch gekennzeichnet, dass** ein Gyroskop integriert ist.

10. Multifunktionale Messvorrichtung nach einem der vorgenannten Ansprüche, **dadurch gekennzeichnet, dass** weitere Sensoren zur Messung des Luftdrucks, der Luftfeuchtigkeit und/oder der Umgebungstemperatur integriert sind.

11. Multifunktionale Messvorrichtung nach einem der vorgenannten Ansprüche, **dadurch gekennzeichnet, dass** Drucksensoren zur Messung des Anpressdrucks des Fingers integriert sind.

12. Multifunktionale Messvorrichtung nach einem der vorgenannten Ansprüche **dadurch gekennzeichnet, dass** Anschlüsse für weitere externe Sensorik am Gehäuse (1) angeordnet sind.

13. Verfahren zur Durchführung einer Messung mit einer multifunktionalen Messvorrichtung nach einem der vorgenannten Ansprüche, **dadurch gekennzeichnet, dass** ein oder mehrere physiologische Parameter durch Ausführung vorgegebener Messprogramme unter Verwendung und/oder Kombination mehrerer Messeinheiten bestimmt werden.

14. Verfahren zur Durchführung einer Messung mit einer multifunktionalen Messvorrichtung nach Anspruch 13, **dadurch gekennzeichnet, dass** aus den Messsignalen weitere Parameter mit statistischen Methoden und/oder Methoden des maschinellen Lernens bestimmt werden, die einer nicht-invasiven Messung sonst nicht zugänglich sind.

## Claims

1. A multifunctional measuring device, comprising a housing (1) having an upper shell (2) and a lower shell (3), which are movable relative to one another by means of a hinge mechanism (4) and comprise cavities which correspond to one another, wherein the cavities form a chamber (9) accessible from the outside for receiving a human finger, wherein an optical measuring unit having an optical module (11), which comprises at least one light source (12) and at least one sensor, is arranged in the chamber (9), and wherein means for data evaluation and/or data transfer are integrated in or on the housing, wherein at least one electrical measuring unit is provided, with at least two measuring electrodes (7) in the chamber (9),
**characterized in that**
on two sides of the lower shell (3), on two outer sides of the measuring device, each are two measuring electrodes for bioimpedance and ECG measurements and on one of the sides additionally a temperature sensor are located.

2. Multifunctional measuring device according to claim 1, **characterized in that** at least one temperature-measuring unit is arranged in and/or on the housing (1).

3. Multifunctional measuring device according to claim 1 or 2, **characterized in that** at least one additional optical sensor (13) and/or one additional light source is arranged in the chamber (9) opposite the optical module (10).

4. Multifunctional measuring device according to any one of the preceding claims, **characterized in that** the hinge mechanism is provided with a return mechanism.

5. Multifunctional measuring device according to any one of the preceding claims, **characterized in that** a microcontroller is arranged in the housing (1) for data evaluation.

6. Multifunctional measuring device according to any one of the preceding claims, **characterized in that** the means for data transfer have a wireless interface.

7. Multifunctional measuring device according to any one of the preceding claims, **characterized in that** devices for positioning the individual fingers are provided such that the fingers are always in the same position during the measuring process.

8. Multifunctional measuring device according to any one of the preceding claims, **characterized in that** an accelerometer is integrated.

9. Multifunctional measuring device according to any one of the preceding claims, **characterized in that** a gyroscope is integrated.

10. Multifunctional measuring device according to any one of the preceding claims, **characterized in that** additional sensors are integrated for measuring the air pressure, humidity and/or the ambient temperature.

11. Multifunctional measuring device according to any one of the preceding claims, **characterized in that** pressure sensors are integrated for measuring the contact pressure of the finger.

12. Multifunctional measuring device according to any one of the preceding claims, **characterized in that** connections for additional external sensor systems are arranged on the housing (1).

13. A method for carrying out a measurement using a multifunctional measuring device according to any one of the preceding claims, **characterized in that** one or more physiological parameters are determined by executing predetermined measuring programs by using and/or combining a plurality of measuring units.

14. Method for carrying out a measurement using a multifunctional measuring device according to claim 13, **characterized in that** additional parameters that are otherwise not accessible to a non-invasive measurement are determined from the measured signals using statistical methods and/or machine-learning methods.

## Revendications

1. Dispositif de mesure multifonctionnel présentant un boîtier (1) avec une coque supérieure (2) et une coque inférieure (3), qui peuvent être déplacées l'une par rapport à l'autre au moyen d'un mécanisme de rabattement (4) et qui présentent des creux correspondants, les creux formant une chambre (9) accessible de l'extérieur pour recevoir un doigt humain, une unité de mesure optique avec un module optique (11), qui présente au moins une source de lumière (12) et au moins un capteur, étant disposée dans la chambre (9) et des moyens pour l'évaluation des données et/ou la transmission des données étant intégrés dans ou sur le boîtier, au moins une unité de mesure électrique étant prévue, avec au moins deux électrodes de mesure (7) dans la chambre (9),
**caractérisé en ce que**
sur deux côtés de la coque inférieure (3), sur deux côtés extérieurs du dispositif de mesure, se trouvent respectivement deux électrodes de mesure (7) pour des mesures de bioimpédance et d'ECG et sur l'un des côtés, en plus, un capteur de température (8).

2. Dispositif de mesure multifonctionnel selon la revendication 1, **caractérisé en ce qu'**au moins une unité de mesure de température est disposée dans et/ou sur le boîtier (1).

3. Dispositif de mesure multifonctionnel selon la revendication 1 ou 2, **caractérisé en ce qu'**au moins un autre capteur optique (13) et/ou une autre source lumineuse sont disposés dans la chambre (9) en face du module optique (10).

4. Dispositif de mesure multifonctionnel selon l'une des revendications précédentes, **caractérisé en ce que** le mécanisme de rabattement est pourvu d'un mécanisme de retour en arrière.

5. Dispositif de mesure multifonctionnel selon l'une des revendications précédentes, **caractérisé en ce qu'**un microcontrôleur est disposé dans le boîtier (1) pour l'évaluation des données.

6. Dispositif de mesure multifonctionnel selon l'une des revendications précédentes, **caractérisé en ce que** les moyens de transmission des données disposent d'une interface sans fil.

7. Dispositif de mesure multifonctionnel selon l'une des revendications précédentes, **caractérisé en ce que** des dispositifs sont prévus pour le positionnement des différents doigts, de telle sorte que les doigts se trouvent toujours dans la même position lors du processus de mesure.

8. Dispositif de mesure multifonctionnel selon l'une des revendications précédentes, **caractérisé en ce qu'**un capteur d'accélération est intégré.

9. Dispositif de mesure multifonctionnel selon l'une des revendications précédentes, **caractérisé en ce qu'**un gyroscope est intégré.

10. Dispositif de mesure multifonctionnel selon l'une des revendications précédentes, **caractérisé en ce que** d'autres capteurs sont intégrés pour mesurer la pression atmosphérique, l'humidité de l'air et/ou la température ambiante.

11. Dispositif de mesure multifonctionnel selon l'une des revendications précédentes, **caractérisé en ce que** des capteurs de pression sont intégrés pour mesurer la pression d'appui du doigt.

12. Dispositif de mesure multifonctionnel selon l'une des revendications précédentes, **caractérisé en ce que** des raccords pour d'autres capteurs externes sont disposés sur le boîtier (1).

13. Procédé pour effectuer une mesure avec un dispositif de mesure multifonctionnel selon l'une des revendications précédentes, **caractérisé en ce qu'**un ou plusieurs paramètres physiologiques sont déterminés par l'exécution de programmes de mesure prédéterminés en utilisant et/ou en combinant plusieurs unités de mesure.

14. Procédé pour effectuer une mesure avec un dispositif de mesure multifonctionnel selon la revendication 13, **caractérisé en ce qu'**à partir des signaux de mesure, d'autres paramètres sont déterminés par des méthodes statistiques et/ou des méthodes d'apprentissage automatique, qui ne sont pas accessibles autrement à une mesure non invasive.
